# EUROPÄISCHE PATENTANMELDUNG

(11) **EP 1 371 627 A1**
(43) Veröffentlichungstag der Anmeldung: **17.12.2003**
(21) Anmeldenummer: 03012420.0
(22) Anmeldetag: 30.05.2003
(51) Int. Cl.: C07C 67/10, C07C 69/157, C07B 41/12, B01J 31/04

(54) **Verfahren zur Herstellung von Carbonsäurebenzylestern**

(30) Priorität: 12.06.2002 DE 10226040
(71) Anmelder: Bayer Aktiengesellschaft, 51368 Leverkusen (DE)
(72) Erfinder: Ooms, Pieter, Dr., 47800 Krefeld (DE); Jansen, Ursula, Dr., 41470 Neuss (DE); Schenke, Bernd-Ulrich, 46236 Bottrop (DE)

(57) **Zusammenfassung**

Carbonsäurebenzylester können durch Umsetzung von Benzylchlorid mit Carbonsäuren in Gegenwart eines oder mehrerer quartären Ammoniumcarboxylate als Katalysator hergestellt werden.

## Beschreibung

Die vorliegende Erfindung betrifft ein Verfahren zur Herstellung von Carbonsäurebenzylestern durch Umsetzung von Benzylchlorid mit Carbonsäuren im Molverhältnis 1 : 1 bis 1: 50 bei 10 bis 200°C und Drucken im Bereich von 0,1 bis 50 bar in Gegenwart eines oder mehrerer quartären Ammoniumcarboxylats als Katalysator.

Benzylacetat, die Hauptkomponente des Jasminöls, ist ein wichtiger Riechstoff zur Herstellung von Duftkompositionen und Ausgangsprodukt für die Herstellung von Fruchtethern.

Die Herstellung von Benzylacetat durch Veresterung von Benzylalkohol mit Essigsäure ist seit langem bekannt.

Benzylacetat kann auch durch Umsetzung von Benzylchlorid mit Alkaliacetaten hergestellt werden (Ullman, Lexikon Chemie, 10. Auflage, Band 2, Seite 1217). Nachteilig ist die Bildung von Salzen, die entsorgt werden müssen und somit die Wirtschaftlichkeit dieser Verfahren verringern.

EP A 0463922 beschreibt die Herstellung von Benzylacetat aus Benzylchlorid und Essigsäure in Gegenwart von tertiären Aminen. Nachteilig ist hierbei, dass äquimolare Mengen Amin eingesetzt werden.

Es bestand die Aufgabe, ausgehend von Benzylchlorid ein Verfahren zur Herstellung von Carbonsäurebenzylestern zu entwickeln, welches unter milden Reaktionsbedingungen durchrührbar ist und kostengünstig zu guten Ausbeuten an Carbonsäurebenzylestern führt.

Es wurde ein Verfahren zur Herstellung von Carbonsäurebenzylestern der Formel 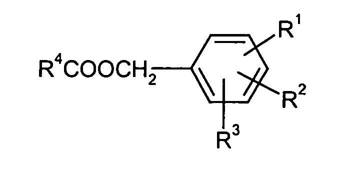 worin
- R¹ bis R³: gleich oder verschieden sind und für Wasserstoff, C₁-C₆-Alkyl, C₁-C₆-Alkoxy-, C₁-C₆-Halogenalkyl, C₁-C₆-Halogenalkoxy-, CN, CO(C₁-C₆-Alkyl), NO₂ oder Halogen und
- R⁴: für Wasserstoff, C₁-C₂₀-Alkyl, C₂-C₂₀-Alkenyl, C₆-C₁₂-Aryl, C₁-C₆-Halogen-alkyl, C₂-C₆-Halogenalkenyl- oder C₆-C₁₂-Halogenaryl,
stehen,
aus Benzylchlorid gefunden, das dadurch gekennzeichnet ist, dass Benzylchloride der Formel 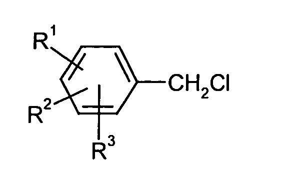 worin
- R¹, R² und R³: die oben genannte Bedeutung haben,
oder Gemische aus Benzylchlorid und Benzylalkoholen der Formel 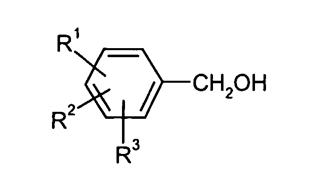 worin
- R¹, R² und R³: die oben genannte Bedeutung haben
mit Carbonsäuren der Formel

R⁴COOH

worin
- R⁴: die oben genannte Bedeutung hat,
in Gegenwart eines oder mehrerer quartären Ammoniumcarboxylats der Formel

R⁴COONR⁵R⁶R⁷R⁸

worin
- R⁵ bis R⁸: gleich oder verschieden sind und für C₁-C₆-Alkyl, C₃-C₆-Cycloalkyl, C₇-C₁₀-Aralkyl-, C₆-C₁₂-Aryl, C₁-C₆-Halogenalkyl-, C₇-C₁₀-Halogenaralkyl- oder C₆-C₁₂-Halogenaryl stehen,
umgesetzt werden.

Das erfindungsgemäße Verfahren lässt sich kostengünstig und unter milden Reaktionsbedingungen durchführen.

Die Reste R¹ bis R³ haben im Allgemeinen die folgende Bedeutung:

Alkyl bedeutet im Allgemeinen ein geradkettiger oder verzweigter Kohlenwasserstoffrest mit 1 bis 6, bevorzugt 1 bis 4, im besondere bevorzugt 1 oder 2 Kohlenstoffatomen. Beispielsweise seien Methyl, Ethyl, Propyl, iso-Propyl, Butyl, iso-Butyl, Pentyl, iso-Pentyl, Hexyl und iso-Hexyl genannt. Bevorzugt sind Methyl, Ethyl, Propyl, iso-Propyl, Butyl, Pentyl und Hexyl, im besondere bevorzugt sind Methyl und Ethyl.

Alkoxy bedeutet im Allgemeinen ein geradkettiger oder verzweigter Alkoxyrest mit 1 bis 6, bevorzugt 1 bis 4, im besondere bevorzugt 1 oder 2 Kohlenstoffatomen. Beispielsweise seien Methoxy, Ethoxy, Propoxy, iso-Propoxy, Butoxy, iso-Butoxy, Pentoxy, iso-Pentoxy, Hexoxy und iso-Hexoxy genannt. Bevorzugt sind Methoxy, Ethoxy, Propoxy, iso-Propoxy, Butoxy, iso-Butoxy, Pentoxy und Hexoxy, im besondere bevorzugt sind Methoxy und Ethoxy.

Halogenalkyl bedeutet im Allgemeinen ein geradkettiger oder verzweigter Kohlenwasserstoffrest mit 1 bis 6, bevorzugt 1 bis 4, im besondere bevorzugt 1 oder 2 Kohlenstoffatomen mit 1 bis 10, bevorzugt 1 bis 8, im besondere bevorzugt mit 1 bis 5 Halogenatomen. Beispielsweise seien Fluormethyl, Difluormethyl, Trifluormethyl, Fluorethyl, Fluorpropyl und Hexafluorbutyl genannt. Bevorzugt sind Fluormethyl, Difluormethyl, Trifluormethyl, Fluorethyl, Fluorpropyl und Hexafluorbutyl, im besondere bevorzugt sind Fluormethyl und Trifluormethyl.

Halogenalkoxy bedeutet im Allgemeinen ein geradkettiger oder verzweigter Alkoxyrest mit 1 bis 6, bevorzugt 1 bis 4, im besondere bevorzugt 1 oder 2 Kohlenstoffatomen mit 1 bis 10, bevorzugt 1 bis 8, im besondere bevorzugt mit 1 bis 5 Halogenatomen. Beispielsweise seien Chlormethoxy, Fluormethoxy, Difluormethoxy, Trifluormethoxy, Fluorethoxy, Fluorpropoxy und Hexafluorbutoxy genannt. Bevorzugt sind Chlormethoxy, Fluormethoxy, Trifluormethoxy, Fluorethoxy, Fluorpropoxy und Hexafluorbutoxy, im besondere bevorzugt sind Fluormethoxy und Trifluormethoxy.

Halogen bedeutet im Allgemeinen Fluor, Chlor, Brom und Jod, bevorzugt Fluor, Chlor und Brom, im besondere Fluor und Chlor.

Ganz besonders bevorzugte Substituenten für R¹ bis R³ sind Wasserstoff, Methyl, Trifluormethyl, Methoxy, Fluor oder Chlor. Ganz besonders bevorzugte Substituenten für R⁴ sind Wasserstoff, Methyl, Ethyl, Propyl, Butyl, Ethenyl, Propenyl, Benzyl, Phenyl, Salicyl oder Styryl.

Nach dem erfindungsgemäßen Verfahren können beispielsweise die folgenden Carbonsäurebenzylestern hergestellt werden:

Ameisensäurebenzylester, Essigsäurebenzylester, Propionsäurebenzylester, Buttersäurebenzylester, Pentansäurebenzylester, Hexansäurebenzylester, Heptansäurebenzylester, Octansäurebenzylester, Nonansäurebenzylester, Decansäurebenzylester, Undecansäurebenzylester, Dodecansäurebenzylester, Tridecansäurebenzylester, Tetradecansäurebenzylester, Pentadecansäurebenzylester, Hexadecansäurebenzylester, Heptadecansäurebenzylester, Oktadecansäurebenzylester, Nonadecansäurebenzylester, 2-Hydroxybenzoesäurebenzylester, 3-Hydroxybenzoesäurebenzylester, 4-Hydroxybenzoesäurebenzylester, 3-Chlor-2-hydroxybenzoesäurebenzylester, 4-Chlor-2-hydroxybenzoesäurebenzylester, 5-Chlor-2-hydroxybenzoesäurebenzylester, 6-Chlor-2-hydroxybenzoesäurebenzylester, 3-Brom-2-hydroxybenzoesäurebenzylester, 3-Fluor-2-hydroxybenzoesäurebenzylester, 4-Fluor-2-hydroxybenzoesäurebenzylester, 2-Fluor-3-hydroxybenzoesäurebenzylester, 2-Fluor-4-hydroxybenzoesäurebenzylester, 3-Fluor-2-hydroxybenzoesäurebenzylester, 2-Fluor-5-hydroxybenzoesäurebenzylester, 2-Fluor-6-hydroxybenzoesäurebenzylester, 2-Hydroxy-3-methylbenzoesäurebenzylester, 2-Hydroxy-4-methylbenzoesäurebenzylester, 3-Hydroxy-2-methylbenzoesäurebenzylester, 4-Hydroxy-2-methylbenzoesäurebenzylester, 2-Fluor-6-hydroxy-4-methoxybenzoesäurebenzylester, 3-Trifluormethyl-2-hydroxybenzoesäurebenzylester, 4-Trifluormethyl-2-hydroxybenzoesäurebenzylester, 2-Trifluormethyl-3-hydroxybenzoesäurebenzylester, 2-Fluorethyl-4-hydroxybenzoesäurebenzylester und 4-Fluorbutyl-2-hydroxybenzoesäurebenzylester.

Benzylchloride für das erfindungsgemäße Verfahren können unsubstituiert oder substituiert sein.

Besonders bevorzugt wird unsubstituiertes Benzylchlorid eingesetzt.

In das erfindungsgemäßen Verfahren können auch Benzylchlorid oder Benzylchlorid /Benzylalkoholgemische, wie sie beispielsweise bei der Herstellung von Benzylalkohol aus Benzylchlorid anfallen, eingesetzt werden. Der Gehalt an Benzylchlorid kann 50 bis 100 %, bevorzugt 60 bis 99 %, besonders bevorzugt 70 bis 98 %, sein.

Bei den im erfindungsgemäßen Verfahren eingesetzten Carbonsäuren handelt es sich um geradkettige und verzweigte, gesättigte und ungesättigte Alkyl-, Aralkyl- und Arylcarbonsäuren mit 1 bis 20 C-Atomen, wie beispielsweise Ameisensäure, Essigsäure, Propionsäure, Buttersäure, Isobuttersäure, Valeriansäure, Isovaleriansäure, Capronsäure, Heptansäure, Caprylsäure, Nonansäure, Caprinsäure, Undecansäure, Laurinsäure, Tridecansäure, Myristinsäure, Palmitinsäure, Stearinsäure, Ölsäure, Linolsäure, Chloressigsäure, Linolensäure, Acrylsäure, Methacrylsäure, Zimtsäure, Phenylessigsäure, Benzoesäure oder Salicylsäure. Bevorzugt sind Carbonsäuren mit 1 bis 15 C-Atomen, besonders bevorzugt 1 bis 10 C-Atomen. Ganz besonders bevorzugte Carbonsäuren sind Ameisensäure, Essigsäure, Chloressigsäure, Propionsäure, Hexansäure und Benzoesäure.

Das erfindungsgemäße Verfahren wird vorzugsweise unter Entfernung des gebildeten Chlorwasserstoffs durchgeführt. Bevorzugt ist die Entfernung des Chlorwasserstoffs durch Durchleiten eines inerten Gases wie beispielsweise Stickstoff.

Geeignete Katalysatoren für das erfindungsgemäße Verfahren sind quartäre Ammoniumcarboxylate wie beispielsweise Tetramethylammoniumformiat, Tetramethylammoniumpropionat, Tetramethylammoniumbutyrat, Tetramethylammoniumbenzoat, Tetramethylammoniumsalicylat, Tetramethylammoniumacetat, Tetraethylammoniumacetat, Tetrapropylammoniumacetat, Tetrabutylammoniumacetat, Benzyltrimethylammoniumacetat, Dibenzyldimethylammoniumacetat, Benzyltriethylammoniumacetat, Benzyltripropylammoniumacetat, Benzyltributylammoniumacetat und Benzyltridodecylammoniumacetat, gegebenenfalls aufgebracht auf einem oder mehreren, bevorzugt einem, Träger.

Bevorzugt sind Tetramethylammoniumacetat, Tetraethylammoniumacetat, Tetrapropylammoniumacetat, Benzyltrimethylammoniumacetat und Benzyltributylammoniumacetat, im besondere bevorzugt sind Tetramethylammoniumacetat und Tetraethylammoniumacetat.

Methoden zur Herstellung der quartären Ammoniumcarboxylate sind bekannt und werden beispielsweise beschrieben in der japanischen Patentanmeldung JP 62.174.036 und US 5 001 156.

Die quartäre Ammoniumcarboxylate, können auf einen Träger aufgebracht, gegebenenfalls kalziniert, als heterogener Katalysator eingesetzt werden.

Die Katalysatoren können z.B. als Pulver oder Formkörper eingesetzt werden und nach der Umsetzung z.B. durch Filtration, Sedimentation oder Zentrifugieren abgetrennt werden.

Für den Fall der Anordnung als Festbett werden die quartäre Ammoniumsalze vorzugsweise auf einem Träger aufgebracht und als Formkörper, Z.B. als Kugeln, Zylinder, Stäbchen, Hohlzylinder, Ringe usw. eingesetzt.

Die quartäre Ammoniumsalzen werden in Mengen von 0,1 bis 100 Gew.-%, bevorzugt von 0,5 bis 90 Gew.-% und besonders bevorzugt von 1,0 bis 80 Gew.-%, bezogen auf Benzylchlorid, verwendet.

Vorzugsweise wird das erfindungsgemäße Verfahren unter intensiver Durchmischung der Reaktionspartner durchgeführt. Eine intensive Durchmischung kann auf verschiedene, dem Fachmann bekannte Weisen, etwa durch Rühren, Düsen, Strombrecher, statische Mischer, Pumpen, turbulente Strömungen in engen Röhren und durch Ultraschall erreicht werden.

Derartige Vorrichtungen sind in Ullmann's Encyclopedia of Industrial Chemistry, 5. Auflage, Band B, Unit Operations, Abschnitte 25, 26, B4 S. 569 bis 572, Verlag Chemie, Weinheim 1988, näher beschrieben.

In einer bevorzugten Ausführungsform des erfindungsgemäßen Verfahrens gibt man in eine Mischung oder Suspension des Benzylchlorids und Carbonsäure quartäres Ammoniumcarboxylat zu und trennt nach Beendigung der Reaktion den Katalysator durch z.B. Filtration oder Zentrifugieren oder nach Isolierung des Carbonsäurebenzylesters ab.

In einer weiteren bevorzugten Ausführungsform des erfindungsgemäßen Verfahrens wird dieses in der Rieselphase durchgeführt und das geträgerte quartäres Ammoniumcarboxylat liegt als Festbettkatalysator vor. Bevorzugt befindet sich die Katalysatorschüttung in einem senkrecht stehenden Rohrreaktor, welcher vorzugsweise Zwischenböden zur besseren Verteilung des Flüssigkeitsstroms und zur besseren Benetzung der Katalysatorschüttung enthält.

Die Aufarbeitung kann sowohl im Falle des suspendierten Katalysators als auch bei Festbettverfahrensvariante so durchgeführt werden, dass ein mit Wasser nicht mischbares Lösungsmittel, vorzugsweise Toluol, zu den Reaktionsprodukten gegeben wird. Nach der Abtrennung der organischen Phase, welche den rohen Carbonsäurebenzylester enthält, kann diese beispielsweise durch Destillation weiter gereinigt werden.

Das erfindungsgemäße Verfahren kann diskontinuierlich, kontinuierlich oder teilkontinuierlich durchgeführt werden.

Die Temperatur, bei der das erfindungsgemäße Verfahren durchgeführt wird, beträgt vorzugsweise 15 bis 200, besonders bevorzugt 25 bis 190, ganz besonders bevorzugt 30 bis 180°C.

Bei der Durchführung der Reaktion oberhalb 115°C muss entsprechend dem Dampfdruck unter erhöhtem Druck gearbeitet werden. Der benötigte Überdruck ist dann wenigstens gleich dem Dampfdruck des Reaktionsgemisches. Es kann bis etwa 200 bar betragen, vorzugsweise bis 50 bar.

Gegebenenfalls kann das erfindungsgemäße Verfahren unter einem üblichen Schutzgas wie beispielsweise Stickstoff, Helium oder Argon, durchgeführt werden.

Das erfindungsgemäße Verfahren kann durch die folgende Reaktionsgleichung erläutert werden:

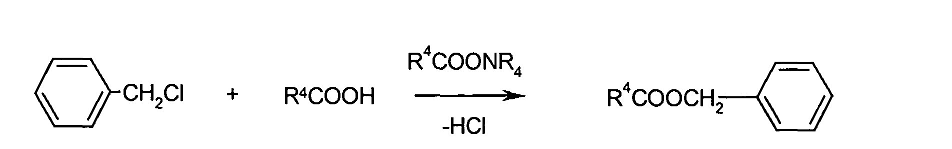

Nach dem erfindungsgemäßen Verfahren erhält man Carbonsäurebenzylester in guten Ausbeuten bei hohem Umsatz und guter Selektivität. Das erfindungsgemäße Verfahren ist ohne hohen apparativen Aufwand einfach durchführbar.

### Beispiele

Die Prozentangaben der nachfolgenden Beispiele beziehen sich auf das Gewicht.

### Beispiel 1

63,3 g (0,5 Mol) Benzylchlorid, 60,0 g (1,0 Mol) Essigsäure und 6,3 g (0,0474 Mol) Tetramethylammoniumacetat wurden in einem Kolben mit Strombrecher und Flügelrührer unter kräftigem Rühren (250 U/min) und unter Stickstoff bei 120°C erhitzt. Nach 3 Stunden Reaktionszeit wurde rasch abgekühlt und das Reaktionsgemisch gaschromatographisch analysiert. Das Reaktionsgemisch enthielt Benzylacetat und Benzylchlorid im Verhältnis 14 zu 85.

### Beispiel 2

Beispiel 1 wurde wiederholt, aber mit 120,0g g (2,0 Mol) Essigsäure. Die Reaktionszeit betrug 34 Stunden. Das Reaktionsgemisch enthielt Benzylacetat und Benzylchlorid im Verhältnis 34 zu 64.

### Beispiel 3

Beispiel 1 wurde wiederholt, aber mit 600,0 g (10,0 Mol) Essigsäure. Die Reaktionszeit betrug 68 Stunden. Das Reaktionsgemisch enthielt Benzylacetat und Benzylchlorid im Verhältnis 70 zu 30.

### Beispiel 4

Beispiel 1 wurde wiederholt, aber mit 6,3 g (0,0241 Mol) Tetraethylammoniumacetat Tetrahydrat eingesetzt. Die Reaktionszeit betrug 7 Stunden. Das Reaktionsgemisch enthielt Benzylacetat und Benzylchlorid im Verhältnis 15 zu 84.

### Beispiel 5

Beispiel 1 wurde wiederholt, aber mit 5,5 g (0,0271 Mol) Tetrapropylammoniumacetat. Die Reaktionszeit betrug 7 Stunden. Das Reaktionsgemisch enthielt Benzylacetat und Benzylchlorid im Verhältnis 15 zu 83.

### Beispiel 6

Beispiel 1 wurde wiederholt, aber mit 6,3 g (0,0209 Mol) Tetrabutylammoniumacetat. Die Reaktionszeit betrug 7 Stunden. Das Reaktionsgemisch enthielt Benzylacetat und Benzylchlorid im Verhältnis 11 zu 88.

### Beispiel 7

Beispiel 1 wurde wiederholt, aber mit 5,1 g (0,0301 Mol) Benzyltrimethylammoniumacetat, hergestellt aus 12,7 g einer wässrigen 40 %-igen Benzyltrimethylammoniumhydroxidlösung, 47,0 g (0,46 Mol) Essigsäureanhydrid und 6,0 g (0,1 Mol) Essigsäure. Die Reaktionszeit betrug 7 Stunden. Das Reaktionsgemisch enthielt Benzylacetat und Benzylchlorid im Verhältnis 14 zu 84.

### Beispiel 8 (Vergleich)

Einsatz eines Tetralkylammoniumchlorids gemäß J.C.S., Chem. Commun., 1991, S.853, 854; EP-A 0 534 817.

Beispiel 1 wurde wiederholt, aber mit 5,2 g (0,0474 Mol) Tetramethylammoniumchlorid. Die Reaktionszeit betrug 3 Stunden. Das Reaktionsgemisch enthielt Benzylacetat und Benzylchlorid im Verhältnis 6 zu 94.

### Beispiel 9 (Vergleich)

Einsatz eines Tetralkylammoniumbromids gemäß J.C.S., Chem. Commun., 1991, S. 853, 854; EP-A 0 534 817.

Beispiel 1 wurde wiederholt, aber mit 7,5 g (0,0474 Mol) Tetramethylammoniumbromid. Die Reaktionszeit betrug 3 Stunden. Das Reaktionsgemisch enthielt Benzylacetat und Benzylchlorid im Verhältnis 10 zu 82.

### Beispiel 10 (Vergleich)

Einsatz eines Benzyldialkylamins gemäß EP-A 0 463 922

Beispiel 1 wurde wiederholt, aber mit 6,3 g (0,0470 Mol) Benzyldimethylamin. Die Reaktionszeit betrug 3 Stunden. Das Reaktionsgemisch enthielt Benzylacetat und Benzylchlorid im Verhältnis 9 zu 81.

## Patentansprüche

1. Verfahren zur Herstellung von Carbonsäurebenzylestem der Formel 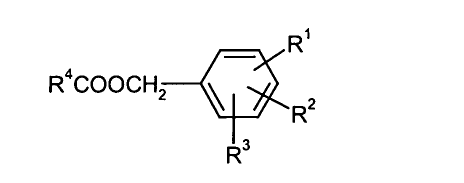 worin
R¹ bis R³ gleich oder verschieden sind und für C₁-C₆-Alkyl, C₁-C₆-Alkoxy-, C₁-C₆-Halogenalkyl, C₁-C₆-Halogenalkoxy-, CN, CO(C₁-C₆-Alkyl), NO₂ oder Halogen und
R⁴ für Wasserstoff, C₁-C₂₀-Alkyl, C₂-C₂₀-Alkenyl, C₆-C₁₂-Aryl, C₁-C₆-Halogenalkyl, C₂-C₆-Halogenalkenyl- oder C₆-C₁₂-Halogenaryl, stehen,
aus Benzylchloriden, **dadurch gekennzeichnet dass** Benzylchloride der Formel 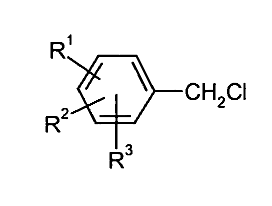 worin
R¹, R² und R³ die oben genannte Bedeutung haben,
oder Gemische aus Benzylchloriden und Benzylalkoholen der Formel 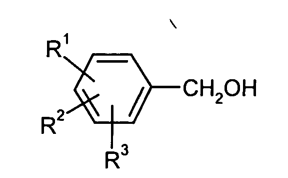 worin
R¹, R² und R³ die oben genannte Bedeutung haben
mit Carbonsäuren der Formel
R⁴COOH
worin
R⁴ die oben genannte Bedeutung hat,
in Gegenwart eines oder mehrerer quartären Ammoniumcarboxylats der Formel
R⁴COONR⁵R⁶R⁷R⁸
worin
R⁵ bis R⁸ gleich oder verschieden sind und für C₁-C₆-Alkyl, C₃-C₆-Cycloalkyl, C₇-C₁₀-Aralkyl-, C₆-C₁₂-Aryl, C₁-C₆-Halogenalkyl-, C₇-C₁₀-Halogenaralkyl- oder C₆-C₁₂-Halogenaryl stehen, umgesetzt werden.

2. Verfahren nach Anspruch 1, **dadurch gekennzeichnet, dass** als quartäres Ammoniumcarboxylat Tetramethylammoniumacetat, Tetraethylammoniumacetat, Tetrapropylammoniumacetat, Tetrabutylammoniumacetat oder Benzyltrimethylammoniumacetat eingesetzt werden.

3. Verfahren nach den Ansprüchen 1 und 2, **dadurch gekennzeichnet, dass** als Carbonsäuren geradkettige und verzweigte, gesättigte und ungesättigte Alkyl -, Aralkyl- und Arylcarbonsäuren mit 1 bis 20 C-Atomen, wie beispielsweise Ameisensäure, Essigsäure, Propionsäure, Buttersäure, Isobuttersäure, Valeriansäure, Isovaleriansäure, Capronsäure, Heptansäure, Caprylsäure, Nonansäure, Caprinsäure, Undecansäure, Laurinsäure, Tridecansäure, Myristinsäure, Palmitinsäure, Stearinsäure, Ölsäure, Linolsäure, Chloressigsäure, Linolensäure, Acrylsäure, Methacrylsäure, Zimtsäure, Phenylessigsäure, Benzoesäure oder Salicylsäure eingesetzt werden.

4. Verfahren gemäß einem oder mehreren der vorangegangenen Ansprüche 1 bis 3, **dadurch gekennzeichnet, dass** es sich bei dem Benzylchlorid um unsubstituiertes Benzylchlorid handelt.

5. Verfahren gemäß einem oder mehreren der vorangegangenen Ansprüche 1 bis 4 **dadurch gekennzeichnet, dass** es sich bei dem Benzylchlorid um ein substituiertes Benzylchlorid handelt, welches einen oder mehrere Substituenten aus der Reihe C₁-C₆-Alkyl, C₁-C₆-Alkoxy-, CN, CO(C₁-C₆-Alkyl), NO₂ oder Halogen trägt.

6. Verfahren gemäß einem oder mehreren der vorangegangenen Ansprüche 1 bis 5, **dadurch gekennzeichnet, dass** 0,1 bis 50 Äquivalente an Carbonsäure, bezogen auf Benzylchlorid, eingesetzt werden.

7. Verfahren gemäß einem oder mehreren der vorangegangenen Ansprüche 1 bis 6, **dadurch gekennzeichnet, dass** 0,1 bis 10 Äquivalente an quartärem Ammoniumcarboxylat, bezogen auf Benzylchlorid, eingesetzt werden.

8. Verfahren gemäß einem oder mehreren der vorangegangenen Ansprüche 1 bis 7, **dadurch gekennzeichnet, dass** die Umsetzung unter Entfernung von Chlorwasserstoff durch Durchleiten von Stickstoff stattfindet.

9. Verfahren gemäß einem oder mehreren der vorangegangenen Ansprüche 1 bis 8, **dadurch gekennzeichnet, dass** die Umsetzung bei einer Temperatur von 15 bis 200°C durchgeführt wird.

10. Verfahren gemäß einem oder mehreren der vorangegangenen Ansprüche 1 bis 9, **dadurch gekennzeichnet, dass** die Umsetzung bei einer Druck von 1 bis 200 bar durchgeführt wird.
